# EUROPÄISCHE PATENTSCHRIFT

(11) **EP 3 762 709 B1**
(45) Veröffentlichungstag und Bekanntmachung des Hinweises auf die Patenterteilung: **13.10.2021**
(21) Anmeldenummer: 19709015.2
(22) Anmeldetag: 04.03.2019
(51) Int. Cl.: G01N 21/82, G01N 21/49, G01N 33/49, G01N 15/02

(54) **VERFAHREN UND ANALYSEVORRICHTUNG ZUM ERMITTELN EINER ANALYTKONZENTRATION IN EINEM FLUID**
METHOD AND ANALYSIS DEVICE FOR DETERMINING AN ANALYTE CONCENTRATION IN A FLUID
PROCÉDÉ ET DISPOSITIF D'ANALYSE POUR LA DÉTERMINATION D'UNE CONCENTRATION D'UN ANALYTE DANS UN FLUIDE

(30) Priorität: 07.03.2018 DE 102018203413
(43) Veröffentlichungstag der Anmeldung: 13.01.2021
(73) Patentinhaber: Anvajo GmbH, 01069 Dresden (DE)
(72) Erfinder: FRAEDRICH, Stefan, 01744 Dippoldiswalde (DE)
(74) Vertreter: Pfenning, Meinig & Partner mbB
(86) Internationale Anmeldenummer: PCT/EP2019/055264
(87) Internationale Veröffentlichungsnummer: WO 2019/170577

(56) Entgegenhaltungen:
- US-A- 5 541 417
- US-A- 5 768 407
- US-A1- 2002 168 784
- US-A1- 2009 287 419
- US-A1- 2009 324 036
- US-A1- 2010 119 132
- US-A1- 2015 369 790
- LI LIN ET AL: "Liquid transparency changing dynamics estimation by means of digital speckle correlation", 2017 PROGRESS IN ELECTROMAGNETICS RESEARCH SYMPOSIUM - SPRING (PIERS), IEEE, 22. Mai 2017 (2017-05-22), Seiten 3172-3175, XP033302238, DOI: 10.1109/PIERS.2017.8262303 [gefunden am 2018-01-16]

## Beschreibung

Die vorliegende Erfindung betrifft ein Verfahren und eine Analysevorrichtung zum Ermitteln einer Analytkonzentration in einem Fluid.

Zum Bestimmen von Analytkonzentrationen in einer Probe gibt es eine Vielzahl von Nachweisverfahren, beispielsweise die Verwendung turbidimetrischer oder nephelometrischer Assays, typischerweise Immunoassays. Diese Assays analysieren Streueigenschaften von sich agglutinierenden Partikeln, oftmals Latexpartikeln. Im Falle von Immunoassays agglutinieren die Partikel meist aufgrund einer Immunreaktion zwischen einem Antigen und einem Antikörper auf einer Oberfläche eines der Partikel. Schwierig an diesen Verfahren ist oftmals eine Weiterverarbeitung der Messwerte, wenn nur geringe Mengen an Probenvolumen zur Verfügung stehen, da Messsignale kaum stärker als ein Hintergrundrauschen sind. Aus der Druckschrift WO 2008/065474 A1 ist beispielsweise ein Verfahren bekannt, bei dem ein Signal-zu-Rausch-Verhältnis durch eine Filterung verbessert wird. Allerdings weist auch ein derartiges Verfahren eine verhältnismäßig große Fehleranfälligkeit auf, da auch mit einer derartigen Filterung in der Regel nur ein schwaches Signal ermittelbar ist.

US 5,541,417 offenbart ein Verfahren zum Analysieren oder Ablesen von Agglutinationsreaktionen wie immunochemischen Reaktionen bei dem die Standardabweichung der Rauheit gegen die AFP-Konzentration (alpha fetal protein) der Probe auf einem zweidimensionalen Graphen aufgetragen wird.

Der vorliegenden Erfindung liegt daher die Aufgabe zugrunde, ein Verfahren und eine Vorrichtung zum Ermitteln einer Analytkonzentration in einem Fluid vorzuschlagen, die die genannten Nachteile vermeiden, mit denen also eine Analytkonzentration mit hoher Genauigkeit ermittelt werden kann.

Diese Aufgabe wird erfindungsgemäß gelöst durch ein Verfahren nach dem unabhängigen Anspruch 1 und eine Analysevorrichtung nach dem unabhängigen Anspruch 6. Vorteilhafte Ausgestaltungen und Weiterbildungen sind in den abhängigen Ansprüchen beschrieben.

Bei einem Verfahren zum Ermitteln einer Analytkonzentration in einem Fluid wird das Fluid, das typischerweise eine Suspension ist, mit dem Analyten und einem turbidimetrischen oder nephelometrischen Assay aus Partikeln in eine Messkammer eingefüllt und die Messkammer von einer Seite mit einer Beleuchtungsvorrichtung beleuchtet. Auf einer der Beleuchtungsvorrichtung abgewandten Seite der Messkammer wird durch mindestens ein Sensorelement eine zweidimensionale Abbildung mit einem von den Partikeln hervorgerufenen Interferenzmuster ortsaufgelöst detektiert. Die detektierte Abbildung wird an eine Recheneinheit weitergeleitet und einer digitalen Bandpassfilterung unterzogen. Nachfolgend wird durch ein Abrastern der Abbildung und Ermitteln einer Standardabweichung jedes Rasterpunkts eine zweidimensionale Abbildung der Standardabweichungen erhalten und an dieser Abbildung der Standardabweichungen eine zweidimensionale Fourier-Transformation durchgeführt. Danach werden die erhaltenen Absolutwerte des durch die Fourier-Transformation generierten Fourier-Spektrums zu einer Summe addiert. Dieser Summe wird durch eine empirisch ermittelte Kalibrierkurve eine Analytkonzentration zugeordnet.

Da mit dem beschriebenen Verfahren zwar eine zweidimensionale Abbildung mit dem von den Partikeln hervorgerufenen Interferenzmuster detektiert wird, dieses Interferenzmuster jedoch nur als vergleichsweise schwaches Signal vorliegt, dienen die nachfolgenden Verfahrensschritte einem Erhöhen einer Genauigkeit des Bestimmens der Analytkonzentration durch eine Signalverarbeitung. Durch die elektronische Bandpassfilterung, die auch als digitale Bandpassfilterung bezeichnet werden kann und von der Recheneinheit durchgeführt wird, können zunächst typischerweise hochfrequente Signalanteile der zweidimensionalen Abbildung, die durch eine Agglutination der Partikel verursacht werden, stärker hervorgehoben werden. Gleichzeitig wird durch den Einsatz der Bandpassfilterung ein Gleichanteil, tiefe Frequenzen und sehr hohe Frequenzen, die durch Rauschen verursacht werden, eliminiert. Durch Berechnen einer lokalen zweidimensionalen Standardabweichung an jedem Punkt des Rasters kann das insgesamt zwar schwache, jedoch homogen über die zweidimensionale Abbildung verteilte Interferenzsignal besser erfasst und der Weiterverarbeitung zugeführt werden. Durch die darauf folgende Fourier-Transformation können die erhaltenen Werte quantifiziert werden, wobei vorzugsweise ein Gleichanteil des Fourier-Spektrums vernachlässigt wird. Die Vernachlässigung des Gleichanteils muss nicht zwangsläufig erfolgen, erlaubt es aber, die eigentlichen Messwerte zuverlässiger zu bestimmen. Indem dieser aufsummierte Wert mit einer empirisch ermittelten Kurve verglichen wird, kann die Analytkonzentration mit hoher Genauigkeit bestimmt werden. Das Verfahren beruht somit im Wesentlichen auf der Idee, nicht wie in konventionellen Verfahren durch eine optische oder digitale Filterung das Signal-zu-Rausch-Verhältnis zu verbessern, sondern stattdessen die spezifische Eigenschaft des erhaltenen Interferenzmusters, nämlich einer homogenen Verteilung über die aufgenommene Abbildung, auszunutzen. Auf kostenintensive Gerätetechnik kann hierbei verzichtet werden, während eine Bildverarbeitung sich auf Schritte beschränkt, die eine sehr robuste und schnelle Interpretation erlauben.

Es kann vorgesehen sein, dass die Bandpassfilterung durch ein Difference-of-Gaussians-Verfahren durchgeführt wird, bei dem zwei Gauß-Funktionen verwendet werden und das eine effiziente und gleichzeitig genaue Filterung ermöglicht.

Die Partikel können als Latexpartikel, Goldpartikel, Silberpartikel, Polymerpartikel, Glaspartikel, Partikel aus einem ferromagnetischen Werkstoff oder biologische Zellen ausgebildet sein, um eine Vielzahl von Untersuchungen durchführen zu können.

Eine mittlere Partikelgröße der Partikel kann zwischen 10 nm und 5 µm, vorzugsweise zwischen 50 nm und 3 µm, besonders vorzugsweise zwischen 100 nm und 2 µm liegen. Diese Größen ergeben bei einer geeigneten Analytkonzentration ein hinreichend gutes Interferenzmuster.

Die Partikel können mit Antikörpern beschichtet sein, um entsprechende Untersuchungen an biologischen Proben durchführen zu können.

Eine Analysevorrichtung zum Ermitteln einer Analytkonzentration in einem Fluid weist eine Messkammer zum Aufnehmen des Fluids mit dem Analyten und einem turbidimetrischen oder nephelometrischen Assay aus Partikeln, eine Beleuchtungsvorrichtung zum Beleuchten der Messkammer, mindestens ein auf einer der Beleuchtungsvorrichtung abgewandten Seite der Messkammer angeordnetes Sensorelement zum ortsaufgelösten Detektieren einer zweidimensionalen Abbildung mit einem von den Partikeln hervorgerufenen Interferenzmuster und einer Recheneinheit auf. Die Recheneinheit ist dazu eingerichtet, die Abbildung einer elektronischen Bandpassfilterung zu unterziehen sowie nachfolgend durch ein Abrastern der Abbildung und Ermitteln einer Standardabweichung jedes Rasterpunkts eine zweidimensionale Abbildung der Standardabweichungen zu erhalten. Die Recheneinheit ist außerdem dazu ausgebildet, an der zweidimensionalen Abbildung der Standardabweichungen eine zweidimensionale Fourier-Transformation durchzuführen und nachfolgend die erhaltenen Absolutwerte des durch die Fourier-Transformation generierten Fourier-Spektrums zu einer Summe zu addieren und durch eine empirisch ermittelte und in der Recheneinheit hinterlegte Kalibrierkurve eine Analytkonzentration der erhaltenen Summe der Absolutwerte zuzuordnen.

Das mindestens eine Sensorelement kann als ein CCD-Sensorelement (chargecoupled device) oder ein CMOS-Sensorelement (complementary metal-oxidesemiconductor) ausgebildet sein.

Die Messkammer kann in einem Abstand zwischen 0 mm und 40 mm zu einer sensitiven Oberfläche des Sensorelements angeordnet sein, um eine möglichst gute Abbildung zu gewährleisten. Die sensitive Oberfläche sollte hierbei der Messkammer zugewandt sein.

Typischerweise sind die Messkammer, die Beleuchtungsvorrichtung und das Sensorelement in einem holographischen optischen Mikroskop angeordnet, so dass sich eine kompakte Anordnung ergibt, mit der schnell und sicher die Analytkonzentration ermittelbar ist.

Das beschriebene Verfahren kann mit der beschriebenen Analysevorrichtung durchgeführt werden, d. h. die beschriebene Analysevorrichtung ist zum Durchführen des beschriebenen Verfahrens ausgebildet.

Ein Computerprogrammprodukt ist zum Durchführen des beschriebenen Verfahrens geeignet. Typischerweise wird das Computerprogrammprodukt zum Steuern der bereits beschriebenen Analysevorrichtung eingesetzt. Das Durchführen des Verfahrens und bzw. oder das Ansteuern der Analysevorrichtung durch das Computerprogrammprodukt erfolgt typischerweise, wenn das Computerprogrammprodukt auf der Recheneinheit abläuft.

Vorzugsweise kann das Computerprogrammprodukt direkt in einen internen Speicher der Recheneinheit geladen werden oder ist in diesem bereits gespeichert und umfasst typischerweise Teile eines Programmcodes zum Durchführen des beschriebenen Verfahrens oder zum Ansteuern der beschriebenen Analysevorrichtung, wenn das Computerprogrammprodukt auf der Recheneinheit abläuft bzw. ausgeführt wird. Das Computerprogrammprodukt kann auf einem maschinenlesbaren Träger, vorzugsweise einem digitalen Speichermedium gespeichert sein. Das Computerprogrammprodukt kann auch ein Computerprogramm umfassen, das Softwaremittel zur Durchführung des beschriebenen Verfahrens und bzw. oder zum Ansteuern der beschriebenen Vorrichtung aufweist, wenn das Computerprogramm in einem Automatisierungssystem oder auf der Recheneinheit ausgeführt wird.

Ausführungsbeispiele der Erfindung sind in den Zeichnungen dargestellt und werden nachfolgend anhand der Figuren 1 bis 5 erläutert.

### Es zeigen:

Fig. 1 eine schematische Ansicht einer Analysevorrichtung mit einer zweidimensionalen Abbildung eines Fluids mit Partikeln;
Fig. 2 eine Figur 1 entsprechende Ansicht mit stärker agglutinierten Partikeln;
Fig. 3 eine Figur entsprechende Ansicht mit einer gegenüber Figur 2 nochmals stärker agglutinierten Partikeln;
Fig. 4 eine Figur 1 entsprechende Ansicht mit zwei Messkammer und
Fig. 5 ein schematischer eines Messvorgangs mit Datenverarbeitung.

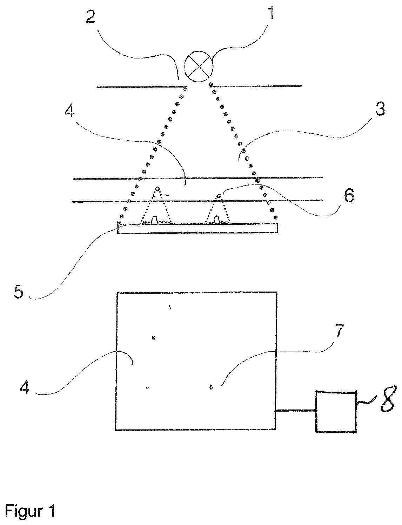

In Figur 1 ist in einer schematischen Ansicht eine Analysevorrichtung dargestellt. Es wird im dargestellten Ausführungsbeispiel eine digitale in-line Holographie verwendet, bei der Hologramme agglutinierter Latexpartikel 6, die in einer Flüssigkeit möglichst gleichmäßig verteilt enthalten sind, abgebildet werden. In weiteren Ausführungsbeispielen können auch Goldpartikel, Silberpartikel, andere Polymerpartikel oder biologische Zellen als turbidimetrische Assays verwendet werden. Die Partikel 6 befinden sich in einer Messkammer 4, die auch als Flusszelle bzw. mikrofluidische Flusszelle bezeichnet wird, die sich in einem Abstand von 0 mm bis 40 mm oberhalb einer sensitiven Oberfläche 7 eines Sensorelements 5 befindet. Das Sensorelement 5 ist im in Figur 1 gezeigten Ausführungsbeispiel ein CMOS-Sensor, kann in weiteren Ausführungsbeispielen aber auch ein CCD-Sensor sein.

Die Messkammer 4 überdeckt im dargestellten Ausführungsbeispiel das Sensorelement 5 und ist fluchtend über ihm angeordnet. Die Messkammer 4 wird außerdem von einer Beleuchtungsvorrichtung 1, die auch als Lichtquelle bezeichnet werden kann, beleuchtet, wobei die von der Beleuchtungsvorrichtung 1 ausgesandte elektromagnetische Strahlung 3 eine Apertur 2 passiert, die zwischen der Beleuchtungsvorrichtung 1 und der Messkammer 4 angeordnet ist. Die Beleuchtungsvorrichtung 1 und das Sensorelement 5 sind dabei einander gegenüberliegend auf verschiedenen Seiten der Messkammer 4 angeordnet, was eine Sonderform einer Anordnung auf einer der Beleuchtungsvorrichtung abgewandten Seite darstellt. Die Messkammer 4 selbst sollte mindestens so groß sein wie das Sensorelement 5 und ist in dem in Figur 1 dargestellten Ausführungsbeispiel mit einer Grundfläche von 50 µm mal 50 µm bis 5 cm mal 5 cm versehen. In weiteren Ausführungsbeispielen kann die Grundfläche auch nicht quadratisch, aber rechteckig sein oder in einer anderen Form vorliegen.Die Apertur 2 weist eine Größe zwischen 1 µm und 500 µm auf und kann auch eine abschließende Fläche bzw. Endfläche einer Glasfaser sein, deren Kerndurchmesser einer Fläche der Apertur 2 entspricht. Ein Abstand zwischen der Beleuchtungsvorrichtung 1 und der sensitiven Oberfläche 7 des Sensorelements 5 beträgt zwischen 1 cm und 10 cm im dargestellten Ausführungsbeispiel. Die Beleuchtungsvorrichtung 1 sendet typischerweise monochromatische elektromagnetische Strahlung im sichtbaren Wellenlängenbereich, d. h. zwischen 400 nm und 780 nm, mit einer FWHM (full width at half maximum) von weniger als 20 nm aus. In weiteren Ausführungsbeispielen kann die Beleuchtungsvorrichtung 1 aber auch eine Weißlichtquelle sein.

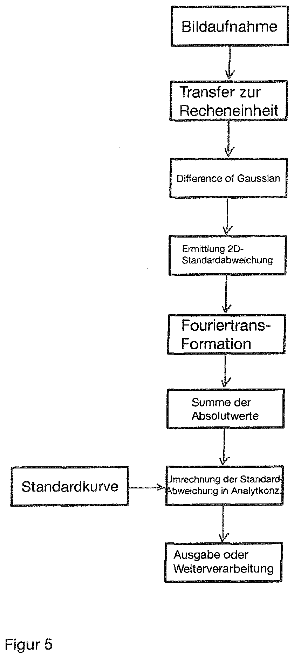

Die Partikel 6 und durch Agglutination aus den Partikeln 6 erhaltene Aggregate werden nicht direkt bildgebend beurteilt, vielmehr werden Interferenzmuster auf der sensitiven Oberfläche 7 des Sensorelements 5 detektiert und einer nur schematisch in Figur 1 gezeigten Recheneinheit 8 zugeführt. Je häufiger und größer die Aggregate der Partikel 6 (eine mittlere Größe der Aggregate beträgt typischerweise zwischen 1 µm und 50 µm, wobei die Partikel 6 selbst in der Regel eine mittlere Partikelgröße zwischen 10 nm und 5 µm aufweisen), desto mehr Interferenzmuster und Interferenzen mit einer höheren Intensität werden auf dem Sensorelement 5 detektiert. Mittels einer Texturanalyse, die im Zusammenhang mit Figur 5 noch genauer erläutert wird, kann ein Zusammenhang zwischen der erhaltenen zweidimensionalen Abbildung auf der sensitiven Oberfläche 7 und einer Analytkonzentration im Fluid ermittelt werden. Während der Aufbau der Analysevorrichtung im oberen Teil von Figur 1 dargestellt ist, wird eine erhaltene zweidimensionale Abbildung im unteren Teil in Draufsicht dargestellt. Die dargestellte turbidimetrische Analysevorrichtung kann auch derart ausgestaltet sein, dass die Messkammer 4, die Beleuchtungsvorrichtung 1 und das Sensorelement 5 in einem einzigen holographischen optischen Mikroskop kombiniert sind, also insbesondere innerhalb eines einzigen Gehäuses angeordnet sind. In einer bevorzugten Ausführungsform ist ein Analyt CRP, also C-reaktives Protein, in Blut, das somit als Probe dient. Das Assay kann hierbei wie beschrieben mit entsprechend beschichteten Latexpartikeln gebildet sein. Die Latexpartikel sind in diesem Fall mit Antikörpern bzw. Antigenen beschichtet, so dass das Agglutinieren durch eine Immunreaktion erfolgt.

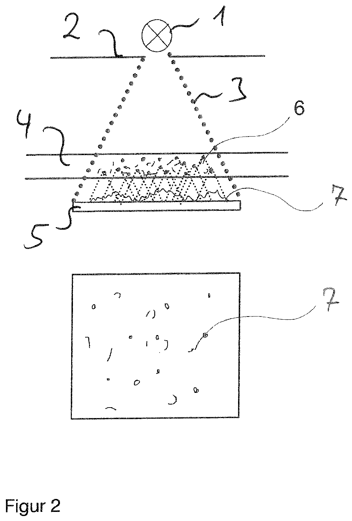

Bei dem in Figur 1 dargestellten Zustand sind die Partikel 6 gut voneinander separiert und erzeugen, je nach Größe daher nur ein sehr schwach ausgeprägtes Interferenzmuster mit geringer Intensität auf der sensitiven Oberfläche 7. In Figur 2 ist in einer Figur 1 entsprechenden Ansicht ein weiteres Ausführungsbeispiel wiedergegeben, bei dem die Partikel gegenüber dem in Figur 1 gezeigten Ausführungsbeispiel stärker agglutiniert sind. Wiederkehrende Merkmale sind in dieser Figur wie auch in den folgenden Figuren mit identischen Bezugszeichen versehen. Bei einer gleichen gerätetechnischen Anordnung fangen die Partikel 6 der Latexpartikelsuspension an, in kleine Cluster zu aggregieren und ein stärker ausgeprägtes Interferenzmuster höherer Intensität entsteht.

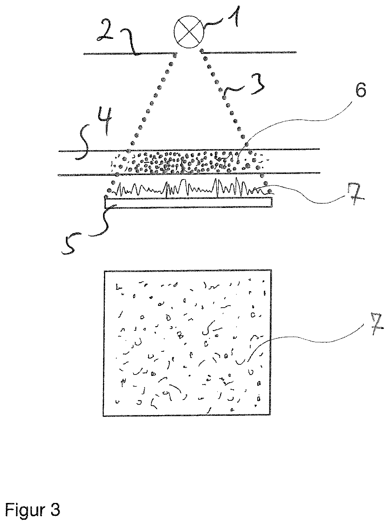

In Figur 3 ist in einer wiederum Figur 1 entsprechenden Ansicht ein Zustand wiedergegeben, bei dem die Partikel gegenüber dem in Figur 2 dargestellten Fall nochmals stärker agglutiniert sind, die Partikelkonzentration selbst jedoch unverändert ist gegenüber den in den Figuren 1 und 2 dargestellten Fällen. Nun sind alle Partikel 6 in Cluster aggregiert und ein sehr stark ausgeprägtes Interferenzmuster ist detektierbar.

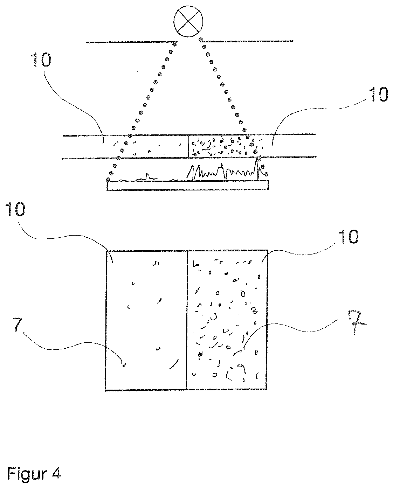

In einer weiteren Ausführungsform, die in Figur 4 in einer wiederum Figur 1 entsprechenden Ansicht dargestellt ist, kann statt einer einzelnen Messkammer 4 auch eine Unterteilung in beliebig viele Untereinheiten 10 der Messkammer 4 erfolgen, die jeweils mit unterschiedlichen Suspensionen gefüllt werden. Die Suspensionen können sich hierbei sowohl hinsichtlich der verwendeten Flüssigkeiten, als auch hinsichtlich der verwendeten Partikel 6 oder der verwendeten Analytkonzentrationen unterscheiden und beliebige Kombinationen sind möglich. Typischerweise werden jedoch bei dem in Figur 4 gezeigten Beispiel mit zwei Untereinheiten 10 der Messkammer 4 Suspensionen verwendet, die sich lediglich in einer Eigenschaft unterscheiden. Im dargestellten Ausführungsbeispiel ist daher die Flüssigkeit und Art der Partikel 6 gleich und lediglich die Analytkonzentration ist unterschiedlich. Die Untereinheiten 10 können einander berühren oder auch voneinander beabstandet angeordnet sein.

Generell erlaubt es diese Konfiguration, verschiedene als Analyten verwendete Suspensionen unabhängig voneinander zu untersuchen. Lediglich beispielhaft gezeigt sind in Figur 4 Interferenzmuster bei einer niedrigen Analytkonzentration im Assay und einer hohen Analytkonzentration im Assay. Dementsprechend sind die Interferenzmuster im rechten Teil stärker ausgeprägt als im linken Teil.. Dies wird durch ein ausreichend großes "field-ofview" erreicht, so dass parallel verschiedene Proben ohne mechanische Bewegung eines Messkopfs untersucht werden können. Die verwendete Sensortechnik samt dem optischen System ist zudem um mindestens eine Größenordnung kleiner als existierende Lösungen zum Auslesen turbidimetrischer Assays, was einen mobilen Einsatz ermöglicht.

Figur 5 stellt ein schematisches Ablaufdiagramm des Messvorgangs und der nachfolgenden Datenverarbeitung dar. Nachdem die Aufnahme der zweidimensionalen Abbildung mit dem Interferenzmuster durch das Sensorelement 5 erfolgt, werden die Daten als digitales Bild an die Recheneinheit 8 transferiert. Als erster Schritt wird ein Difference-of-Gaussians-Verfahren auf die Abbildung angewendet, das als elektronischer bzw. digitaler Bandpassfilter wirkt. Parameter der beiden hierfür verwendeten Gauß-Funktionen sind derart gewählt, dass hochfrequente Signalanteile, die durch die Agglutination der Partikel 6 verursacht werden, dominant hervorgehoben werden. Beispielsweise werden folgende Parameter benutzt: Gauß-Puls 1 hat eine Fensterbreite von 3,95 Prozent der gesamten Abbildung, also beträgt die Gauß-Breite bei einer Bildgröße von 2048 mal 2048 Pixeln gerade 81 mal 81 Pixel. Als Sigma kann 0,1 bis 0,000001 (der letztere Wert bei geringem Rauschen) verwendet werden. Die zweite Gauß-Funktion kann die gleiche Fenstergröße, aber ein größeres Sigma von beispielsweise 5 aufweisen. Dieser Bandpassfilter eliminiert einen Gleichanteil und tiefe Frequenzen sowie sehr hohe Frequenzen, die durch Rauschen verursacht werden. Die Parameter der Gauß-Funktionen, die "Grenzbedingungen" festlegen, werden empirisch gefunden.

Aus der mittels dem Difference-of-Gaussians-Verfahren überarbeiteten Abbildung wird eine lokale zweidimensionale Standardabweichung ermittelt, indem die Abbildung mit Fenstern von 3 mal 3 Pixeln Größe oder 6 mal 6 Pixeln Größe abgerastert wird und für jedes Bild bzw. Fenster die Standardabweichung berechnet wird. Hierbei wird ausgenutzt, dass die Interferenz zwar in der Regel schwach ausgeprägt, aber dafür homogen verteilt ist. Die so erhaltene zweidimensionale Abbildung der Standardabweichungen wird einer zweidimensionalen Fourier-Transformation unterzogen. Die Absolutwerte des Fourier-Spektrums werden aufsummiert, wobei ein Gleichanteil nicht mit in die Summe eingeht und stattdessen verworfen wird. Über eine empirisch mittels bekannter Analytkonzentrationen durch Regression ermittelte Standardkurve kann dann die Konzentration der Partikel 6 ermittelt und von der Recheneinheit 8 weiterverarbeitet und bzw. oder auf einer Ausgabeeinheit wie einem Bildschirm angezeigt werden. Diese Standard- oder Kalibrierkurve kann in einer Speichereinheit der Recheneinheit 8 hinterlegt sein und bei Bedarf aufgerufen werden. Somit kann über das beschriebene Verfahren und die beschriebene Analysevorrichtung ein Quantifizieren der Partikelagglutination 6 erreicht werden.

## Patentansprüche

1. Verfahren zum Ermitteln einer Analytkonzentration in einem Fluid, bei dem
in eine Messkammer (4) das Fluid mit dem Analyten und einem turbidimetrischen oder nephelometrischen Assay aus Partikeln (6) eingefüllt wird,
die Messkammer (4) von einer Seite mit einer Beleuchtungsvorrichtung (1) beleuchtet wird und
auf einer der Beleuchtungsvorrichtung (1) abgewandten Seite der Messkammer (4) durch mindestens ein Sensorelement (5) eine zweidimensionale Abbildung mit einem von den Partikeln (6) hervorgerufenen Interferenzmuster ortsaufgelöst detektiert wird, wobei
die detektierte Abbildung an eine Recheneinheit (8) weitergeleitet wird und durch die Recheneinheit (8) einer elektronischen Bandpassfilterung unterzogen wird
sowie nachfolgend ein Abrastern der Abbildung und eine Festlegung von einer Vielzahl von Fenstern jeweils umfassend eine Vielzahl von Pixeln durchgeführt wird, wobei für jedes Fenster eine lokale Standardabweichung ermittelt wird, derart, dass eine zweidimensionale Abbildung von Standardabweichungen erhalten wird, und
an dieser zweidimensionalen Abbildung der Standardabweichung eine zweidimensionale Fourier-Transformation durchgeführt wird und
nachfolgend die erhaltenen Absolutwerte des durch die Fourier-Transformation generierten Fourier-Spektrums zu einer Summe addiert werden sowie
durch eine empirisch ermittelte Kalibrierkurve der erhaltenen Summe der Absolutwerte eine Analytkonzentration zugeordnet wird.

2. Verfahren nach Anspruch 1, **dadurch gekennzeichnet, dass** die Bandpassfilterung durch ein Difference of Gaussians-Verfahren durchgeführt wird.

3. Verfahren nach Anspruch 1 oder Anspruch 2, **dadurch gekennzeichnet, dass** die Partikel (6) als Latexpartikel, Goldpartikel, Silberpartikel, Polymerpartikel, Glaspartikel, Partikel aus ferromagnetischen Werkstoffen oder biologische Zellen ausgebildet sind.

4. Verfahren nach einem der vorhergehenden Ansprüche, **dadurch gekennzeichnet, dass** die Partikel (6) eine mittlere Partikelgröße zwischen 10 nm und 5 µm, vorzugsweise zwischen 50 nm und 3 µm, besonders vorzugsweise zwischen 100 nm und 2 µm, aufweisen.

5. Verfahren nach einem der vorhergehenden Ansprüche, **dadurch gekennzeichnet, dass** die Partikel (6) mit Antikörpern beschichtet sind.

6. Analysevorrichtung zum Ermitteln einer Analytkonzentration in einem Fluid, mit
einer Messkammer (4) zum Aufnehmen des Fluids mit dem Analyten und einem turbidimetrischen oder nephelometrischen Assay aus Partikeln (6),
einer Beleuchtungsvorrichtung (1) zum Beleuchten der Messkammer (4),
mindestens einem auf einer der Beleuchtungsvorrichtung (1) abgewandten Seite der Messkammer (4) angeordneten Sensorelement (5) zum ortsaufgelösten Detektieren einer zweidimensionalen Abbildung mit einem von den Partikeln (6) hervorgerufenen Interferenzmuster, einer Recheneinheit (8), die eingerichtet ist, die Abbildung einer elektronischen Bandpassfilterung zu unterziehen
sowie nachfolgend ein Abrastern der Abbildung und eine Festlegung von einer Vielzahl von Fenstern jeweils umfassend eine Vielzahl von Pixeln durchzuführen und für jedes Fenster eine lokale Standardabweichung zu ermitteln, derart, dass eine zweidimensionale Abbildung von Standardabweichungen erhalten wird, und an der zweidimensionalen Abbildung der Standardabweichung eine zweidimensionale Fourier-Transformation durchzuführen sowie
nachfolgend die erhaltenen Absolutwerte des durch die Fourier-Transformation generierten Fourier-Spektrums zu einer Summe zu addieren und durch eine empirisch ermittelte Kalibrierkurve der erhaltenen Summe der Absolutwerte eine Analytkonzentration zuzuordnen.

7. Analysevorrichtung nach Anspruch 6, **dadurch gekennzeichnet, dass** das Sensorelement (5) ein CCD-Sensorelement oder ein CMOS-Sensorelement ist.

8. Analysevorrichtung nach einem der Ansprüche 6 oder 7, **dadurch gekennzeichnet, dass** die Messkammer (4) in einem Abstand zwischen 0 mm und 40 mm zu einer sensitiven Oberfläche (7) des Sensorelements (5) angeordnet ist.

9. Analysevorrichtung nach einem der Ansprüche 6 bis 8, **dadurch gekennzeichnet, dass** die Messkammer (4), die Beleuchtungsvorrichtung (1) und das Sensorelement (5) in einem holographischen optischen Mikroskop angeordnet sind.

10. Computerprogramm, umfassend Befehle, die bewirken, dass die Vorrichtung nach einem der Ansprüche 6-9 die Verfahrensschritte nach einem der Ansprüche 1-5 ausführt.

## Claims

1. Method for determining an analyte concentration in a fluid, in which
the fluid with the analyte and a turbidimetric or nephelometric assay of particles (6) is filled into a measuring chamber (4),
the measuring chamber (4) is illuminated from one side with an illumination device (1), and
on a side of the measuring chamber (4) orientated away from the illumination device (1), a two-dimensional image with an interference pattern caused by the particles (6) is detected, spatially resolved, by at least one sensor element (5),
the detected image being further conducted to a computer unit (8) and subjected to an electronic bandpass filtering by the computer unit (8),
and subsequently scanning of the image and establishing a large number of windows respectively comprising a large number of pixels is implemented, for each window, a local standard deviation being determined, such that a two-dimensional image of standard deviations is obtained, and
a two-dimensional Fourier transformation is implemented on this two-dimensional image of the standard deviation, and
subsequently the obtained absolute values of the Fourier spectrum generated by the Fourier transformation are added to form a sum,
and an analyte concentration is assigned by an empirically determined calibration curve to the obtained sum of the absolute values.

2. Method according to claim 1, **characterised in that** the bandpass filtering is implemented by a difference of Gaussians method.

3. Method according to claim 1 or claim 2, **characterised in that** the particles (6) are formed as latex particles, gold particles, silver particles, polymer particles, glass particles, particles made of ferromagnetic materials or biological cells.

4. Method according to one of the preceding claims, **characterised in that** the particles (6) have an average particle size between 10 nm and 5 µm, preferably between 50 nm and 3 µm, particularly preferably between 100 nm and 2 µm.

5. Method according to one of the preceding claims, **characterised in that** the particles (6) are coated with antibodies.

6. Analysis device for determining an analyte concentration in a fluid, having
a measuring chamber (4) for receiving the fluid with the analyte and a turbidimetric or nephelometric assay of particles (6),
an illumination device (1) for illuminating the measuring chamber (4),
at least one sensor element (5), disposed on a side of the measuring chamber (4) orientated away from the illumination device (1), for spatially resolved detection of a two-dimensional image with an interference pattern caused by the particles (6),
a computer unit (8) which is fitted to subject the image to an electronic bandpass filtering,
and subsequently to implement scanning of the image and establishing a large number of windows respectively comprising a large number of pixels, and for each window, to determine a local standard deviation, such that a two-dimensional image of standard deviations is obtained, and to implement a two-dimensional Fourier transformation on the two-dimensional image of the standard deviation, and
subsequently to add the obtained absolute values of the Fourier spectrum generated by the Fourier transformation to form a sum, and to assign an analyte concentration by an empirically determined calibration curve to the obtained sum of the absolute values.

7. Analysis device according to claim 6, **characterised in that** the sensor element (5) is a CCD sensor element or a CMOS sensor element.

8. Analysis device according to one of claims 6 or 7, **characterised in that** the measuring chamber (4) is disposed at a spacing between 0 mm and 40 mm relative to a sensitive surface (7) of the sensor element (5).

9. Analysis device according to one of claims 6 to 8, **characterised in that** the measuring chamber (4), the illumination device (1) and the sensor element (5) are disposed in a holographic optical microscope.

10. Computer program comprising commands which cause the device according to one of claims 6 ― 9 to implement the method steps according to one of claims 1 ― 5.

## Revendications

1. Procédé de détermination d'une concentration en analyte dans un fluide, chez lequel
le fluide avec l'analyte et un milieu de dosage turbidimétrique ou néphélométrique à base de particules (6) est introduit dans une chambre de mesure (4),
la chambre de mesure (4) est éclairée à partir d'un côté avec un dispositif d'éclairage (1), et
une représentation bidimensionnelle avec un motif interférentiel créé du fait des particules (6) est détectée avec une résolution spatiale sur un côté de la chambre de mesure (4) opposé au dispositif d'éclairage (1) par au moins un élément capteur (5), où
la représentation détectée est transmise à une unité de calcul (8) et est soumise à un filtrage passe-bande électronique par l'unité de calcul (8)
de même, ensuite, on effectue un balayage de la représentation et on détermine un grand nombre de fenêtres comprenant respectivement un grand nombre de pixels, où, un écart-type local est déterminé pour chaque fenêtre, de telle manière qu'une représentation bidimensionnelle d'écart-types est obtenue, et
on effectue une transformation de Fourier bidimensionnelle sur cette représentation bidimensionnelle de l'écart-type, et
ensuite, les valeurs absolues obtenues par le spectre de Fourier généré par la transformation de Fourier sont additionnées pour donner une somme, de même,
une concentration en analyte est associée à la somme des valeurs absolues obtenue par une courbe d'étalonnage de déterminée de manière empirique.

2. Procédé selon la revendication 1, **caractérisé en ce que** le filtrage passe-bande est exécuté par un procédé de différence de gaussiennes.

3. Procédé selon la revendication 1 ou la revendication 2, **caractérisé en ce que** les particules (6) sont conçues sous forme de particules de latex, de particules d'or, de particules d'argent, de particules de polymères, de particules de verre, de particules à base de matériaux ferromagnétiques ou de cellules biologiques.

4. Procédé selon l'une des revendications précédentes, **caractérisé en ce que** les particules (6) présentent une taille particulaire moyenne entre 10 nm et 5 µm, de préférence entre 50 nm et 3 µm, de manière particulièrement préférée, entre 100 nm et 2 µm.

5. Procédé selon l'une des revendications précédentes, **caractérisé en ce que** les particules (6) sont revêtues d'anticorps.

6. Dispositif d'analyse pour la détermination d'une concentration en analyte dans un fluide, avec
une chambre de mesure (4) pour recevoir le fluide avec l'analyte et un milieu de dosage turbidimétrique ou néphélométrique à base de particules (6),
un dispositif d'éclairage (1) pour éclairer la chambre de mesure (4),
au moins un élément capteur (5) disposé sur un côté de la chambre de mesure (4) opposé au dispositif d'éclairage (1) pour la détection avec une résolution spatiale d'une représentation bidimensionnelle avec un motif interférentiel du fait des particules (6),
une unité de calcul (8), qui est conçue pour soumettre la représentation à un filtrage passe-bande électronique,
de même pour, ensuite, effectuer un balayage de la représentation et déterminer un grand nombre de fenêtres comprenant respectivement un grand nombre de pixels et pour déterminer un écart-type local pour chaque fenêtre, de telle manière qu'une représentation bidimensionnelle d'écart-types est obtenue, et pour effectuer une transformation de Fourier bidimensionnelle sur cette représentation bidimensionnelle de l'écart-type, de même,
pour, ensuite, additionner les valeurs absolues obtenues par le spectre de Fourier généré par la transformation de Fourier pour donner une somme, et pour associer une concentration en analyte à la somme des valeurs absolues obtenue par une courbe d'étalonnage déterminée de manière empirique.

7. Dispositif d'analyse selon la revendication 6, **caractérisé en ce que** l'élément détecteur (5) est un élément détecteur CCD ou un élément détecteur CMOS.

8. Dispositif d'analyse selon l'une des revendications 6 ou 7, **caractérisé en ce que** la chambre de mesure (4) est disposée à une distance entre 0 mm et 40 mm par rapport à une surface sensible (7) de l'élément détecteur (5).

9. Dispositif d'analyse selon l'une des revendications 6 à 8, **caractérisé en ce que** la chambre de mesure (4), le dispositif d'éclairage (1) et l'élément détecteur (5) sont disposés dans un microscope optique holographique.

10. Programme informatique, comprenant des commandes qui font en sorte que le dispositif selon l'une des revendications 6 à 9 exécute les étapes de procédé selon l'une des revendications 1 à 5.
